# FASCICULE DE BREVET EUROPEEN

(11) **EP 0 394 111 B1**
(45) Date de publication et mention de la délivrance du brevet: **04.06.1997**
(21) Numéro de dépôt: 90401020.4
(22) Date de dépôt: 13.04.1990
(51) Int. Cl.: C07K 5/06, C07F 9/10

(54) **Nouvelles lipopolyamines, leur préparation et leur emploi**
Lipopolyamine, deren Herstellung und Verwendung
Lipopolyamines, their preparation and use

(30) Priorité: 17.04.1989 FR 8905037
(43) Date de publication de la demande: 24.10.1990
(73) Titulaire: CENTRE NATIONAL DE LA RECHERCHE SCIENTIFIQUE (CNRS), 75700 Paris Cedex 07 (FR)
(72) Inventeur: Behr, Jean-Paul, F-67100 Strasbourg (FR); Loeffler, Jean-Philippe, F-67000 Strasbourg (FR)
(74) Mandataire: Pilard, Jacques

(56) Documents cités:
- TETRAHEDRON LETTERS, vol. 27, no. 48, 1986, pages 5861-5864, Pergamon Journals Ltd, Oxford, GB; J.-P. BEHR: "DNA strongly binds to micelles and vesicles containing lipopolyamines or lipointercalants"
- PROCEEDINGS OF NATIONAL ACADEMY OF SCIENCES OF USA, vol. 86, no. 18, septembre 1989, pages 6982-6986; J.-P. BEHR et al.: "Efficient gene transfer into mammalian primary endocrine cells with lipopolyamine-coated DNA"
- CA,vol.104, no. 18681
- Tetr. Letters, vol. 30, no. 11, pp. 1401-1404
- Synthesis, 1986, pp. 303-306
- Tetr. Letters, vol. 44, no. 6, pp. 1773-1782
- Tetr. Letters, vol. 37, no. 17, pp. 3037-3041

## Description

La présente invention concerne de nouvelles lipopolyamines de formule générale : 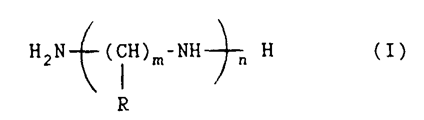 sous forme D, L ou DL, leurs sels, leur préparation et leur emploi.

Dans la formule générale (I),
- n est un nombre entier compris entre 1 et 5 inclusivement,
- m est un nombre entier compris entre 2 et 6 inclusivement,
- R représente un atome d'hydrogène ou un radical de formule générale: 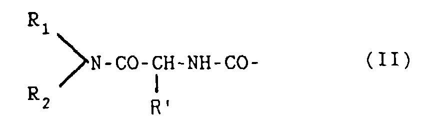 dans lequel R₁ et R₂, identiques ou différents, représentent chacun un radical aliphatique saturé CₚH₂ₚ₊₂ ou insaturé CₚH₂ₚ ou CₚH₂ₚ₋₂, p étant un nombre entier compris entre 12 et 22 inclusivement, et R' représente un atome d'hydrogène ou un radical alkyle contenant 1 à 4 atomes de carbone éventuellement substitué par un radical phényle, ou un radical de formule générale : 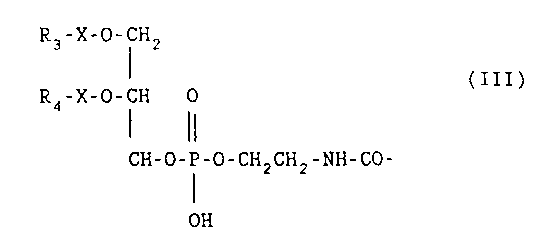 dans lequel X représente un groupement méthylène (-CH₂-) ou un groupement carbonyle (-CO-), et R₃ et R₄, identiques ou différents, représentent chacun un radical aliphatique saturé C_{p'}H_{2p'+2} ou insaturé C_{p'}H_{2p'} ou C_{p'}H_{2p'-2}, p étant un nombre entier compris entre 11 et 21 inclusivement, étant entendu que :
   - quelles que soient les valeurs de m et n, un et un seul des symboles R représente un radical de formule générale (II) ou (III)
   - lorsque n est compris entre 2 et 5, les valeurs de m dans les différents fragments 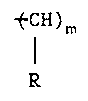 peuvent être identiques ou différentes.

D'un intérêt tout particulier sont les produits de formule générale (I) dans laquelle n est égal à 3 et les valeurs de m dans les fragments 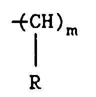 sont identiques ou différentes et représentent 3 ou 4, et R représente :
soit un radical de formule générale (II) dans lequel R₁ et R₂ représentent chacun un radical alkyle contenant 12 à 22 atomes de carbone et R' représente un atome d'hydrogène,
soit un radical de formule générale (III) dans lequel R₃-X- et R₄-X-représentent chacun un radical alcanoyle contenant 12 à 22 atomes de carbone.

Plus particulièrement intéressants encore sont la 5-carboxyspermylglycine dioctadécylamide (DOGS) et 5-carboxyspermylamide de la dipalmitoylphosphatidyléthanolamine (DPPES).

Selon l'invention, les nouvelles lipopolyamines de formule générale (I) peuvent être obtenues par action sur un produit de formule générale : 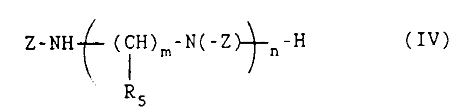 dans laquelle m et n sont définis comme précédemment, R₅ représente un atome d'hydrogène ou un radical carboxy et les symboles Z représentent un groupement protecteur de la fonction amine, étant entendu que :
- quelles que soient les valeurs de m et n, un et un seul des symboles R₅ représente un radical carboxy
- lorsque n est compris entre 2 et 5, les valeurs de m des différents fragments 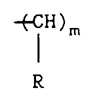 peuvent être identiques ou différentes soit d'un produit de formule générale : 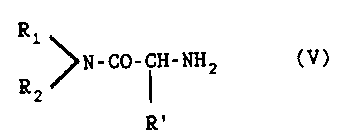 dans laquelle R₁, R₂ et R' sont définis comme précédemment,
   soit d'un produit de formule générale : 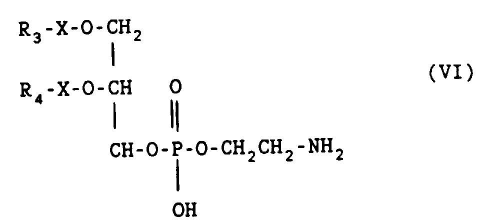 dans laquelle R₃, R₄ et X sont définis comme précédemment, suivie du remplacement des groupements protecteurs Z par un atome d'hydrogène.

Lorsque l'on fait réagir un produit de formule générale (V) sur un produit de formule générale (IV), il est particulièrement avantageux d'effectuer la condensation en présence d'un diimide tel que le dicyclohexylcarbodiimide en opérant dans un solvant organique inerte choisi parmi les solvants aliphatiques halogénés tels que le chlorure de méthylène.

Lorsque l'on fait réagir un produit de formule générale (VI) sur un produit de formule générale (IV), il est particulièrement avantageux de traiter préalablement la fonction acide du produit de formule générale (IV) par le N-hydroxysuccinimide en opérant dans un solvant organique choisi parmi les hydrocarbures aliphatiques halogénés (chlorure de méthylène) et les éthers (tétrahydrofuranne) en présence d'un imide tel que le dicyclohexylcarbodiimide, avant d'effectuer la condensation du produit de formule générale (VI). La condensation de l'ester mixte sur le produit de formule générale (VI) s'effectue généralement dans un solvant organique (chloroforme, éthanol) en présence d'une base organique telle que la triéthylamine à une température comprise entre 30 et 50°C.

Généralement, on utilise un groupement protecteur Z qui est facilement remplaçable par un atome d'hydrogène sans toucher au reste de la molécule. Il est particulièrement avantageux d'utiliser comme groupement protecteur le radical t.butoxycarbonyle qui est facilement remplaçable par un atome d'hydrogène au moyen d'un acide (acide trifluoroacétique).

Les produits de formule générale (IV) dans laquelle n est supérieur à 1 peuvent être obtenus à partir de l'ornithine par cyanoalkylation suivie de réduction des fonctions nitriles en fonctions amines, puis protection des fonctions amines ainsi obtenues.

Les produits de formule générale (V) peuvent être obtenus par action d'une amine de formule générale : 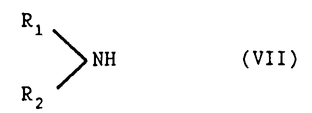 dans laquelle R₁ et R₂ sont définis comme précédemment, sur un aminoacide de formule générale : 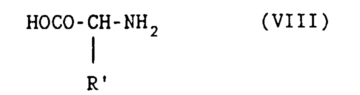 dans laquelle R' est défini comme précédemment, dont la fonction amine est protégée et dont la fonction acide est activée. Il est particulièrement avantageux de protéger la fonction amine par un radical benzyloxycarbonyle qui est facilement remplaçable par un atome d'hydrogène par hydrogénolyse en présence d'un catalyseur tel que le palladium. Généralement, la fonction acide est activée par transformation en ester p.nitrophénylé.

Les produits de formule générale (VI) sont des produits connus qui sont facilement accessibles.

Un certain nombre de techniques ont été developpées pour introduire de l'ADN dans des cellules eucaryotiques. En particulier, une approche a consisté à lier l'ADN à la couche externe de liposomes ou de micelles, en le complexant à la partie cationique de lipopolyamines ou de lipointercalants, ancrés par leur partie hydrophobe à ces vésicules ( Behr J.-P., Tetrahedron Letters, Vol. 27, No 48, pp 5861-5864, 1986).Les vésicules ainsi formées évoquent des nucléosomes.

Les nouveaux produits de formule générale (I) présentent la propriété, dispersés dans l'eau, de former des nanoparticules unilamellaires, qui sont instables en milieu ionique, et qui s'associent fortement, par leur partie cationique, avec l'ADN plasmidique ou oligonucléotidique en le compactant et en le recouvrant d'une couche lipidique. En utilisant un excès de charges cationiques par rapport à l'acide nucléique, les complexes lipide/ADN peuvent être adsorbés sur les membranes cellulaires facilitant ainsi la capture de l'ADN par les cellules.

Les produits de formule générale (I) constituent des vecteurs spécifiques, non toxiques, biodégradables et de grande efficacité pour réaliser la transfection de cellules eucaryotes (lignées cellulaires, cultures primaires).

Selon l'invention, la transfection est réalisée en mettant en contact une suspension de cellules en absence de sérum avec un mélange transfectant obtenu, au moment de sa mise en oeuvre, à partir d'une solution de la lipopolyamine de formule générale (I) et d'une solution de l'ADN dans un milieu convenable.

Il est particulièrement avantageux d'opérer en milieu très dilué (1 à 5 nanomolaire) et d'utiliser un excès (de 2 à 5 fois) de charges de la lipopolyamine par rapport à l'ADN.

La durée de la transfection peut être comprise entre 10 minutes et 48 heures indépendamment de la nature des cellules.

Le procédé selon l'invention présente l'avantage de pouvoir s'appliquer à des lignées cellulaires d'origines diverses (incluant par exemple LMKT, Ras4, CHO, F9, Bu4, S49, Hela et AtT20) ainsi qu'à des cellules primaires sans qu'il soit nécessaire d'optimiser ou de modifier les conditions de mise en oeuvre du procédé.

Par ailleurs, les lipopolyamines de formule générale (I) permettent de transfecter des cellules fragiles (cellules hypophysaires intermédiaires ou antérieures, cellules chromaffin, neurones périphériques ou centraux) qu'il n'était pas possible de transfecter par application de méthodes classiques (coprécipitation au phosphate de calcium ou les techniques au dextrane).

Enfin, les agents de transfection selon l'invention ne manifestent pas de toxicité vis-à-vis des cellules transfectées. Ils ne manifestent pas de toxicité aiguë chez le rat après injection intracérébrale ou systémique.

La présente invention a pour objet également une solution alcoolique ou aqueuse stable d'une lipopolyamine de formule générale (I) utilisable pour la réalisation de transfections cellulaires.

Généralement, on prépare des solutions à 1 mg/ml qui permettent la réalisation d'environ 50 transfections.

Les exemples suivants, donnés à titre non limitatif, montrent comment l'invention peut être mise en pratique.

### EXEMPLE 1

On agite pendant 12 heures un mélange de L-5-carboxy-tétra-t.butoxycarbonylspermine (1 équivalent) et de glycine dioctadécylamide (1 équivalent) dans le chlorure de méthylène en présence de dicyclohexylcarbodiimide (1,1 équivalent).

Après chromatographie sur silice, on obtient, avec un rendement de 90 %, la tétra-t.butoxycarbonyl-5-carboxyspermylglycine dioctadécylamide dont les groupements protecteurs sont éliminés par traitement par l'acide trifluoroacétique pendant 10 minutes à une température voisine de 20°C. On obtient ainsi le tétratrifluoroacétate de 5-carboxyspermylglycine dioctadécylamide (DOGS).

La structure du produit obtenu est confirmée par le spectre de résonance magnétique nucléaire du proton à 200 MHz dans le méthanol deutéré (déplacements chimiques δ en ppm) : 0,9 [t, (CH₃)₂] ; 1,3 [m, 2 x (CH₂)₁₅] ; 1,4-1,7 (m, 2 x CH₂CH₂NCO) ; 1,8-2,2 (m, 4 x CH₂CH₂N⁺) ; 3,0-3,2 (m, 5 x CH₂N⁺) ; 3,35 (t, 2 x CH₂NCO) ; 4,0 (t, CHN⁺) ; 4,15 (s, COCH₂ND).

La L-5-carboxy-tétra-t.butoxycarbonylspermine peut être préparée de la manière suivante :

A une solution 1M de L-ornithine dans le diméthylformamide, on ajoute 2,2 équivalents d'acrylonitrile. On agite pendant 1 heure à une température voisine de 20°C.

Le dinitrile ainsi obtenu est réduit par l'hydrogène en présence de nickel de Raney en opérant en présence de potasse éthanolique pour donner la L-5-carboxyspermine dont les fonctions amines sont protégées par des groupements t.butoxycarbonyle par application des méthodes habituelles.

La glycinedioctadécylamide peut être préparé par condensation de dioctadécylamine (1 équivalent) avec l'ester p.nitrophényle de la N-carbobenzoxy-glycine (1 équivalent) en opérant dans le chlorure de méthylène en présence de triéthylamine (1,1 équivalent) pendant 5 heures.

Après hydrogénation pendant 1 heure en présence de palladium sur noir à 10 % en opérant dans un mélange chlorure de méthylène-éthanol, on obtient la glycinedioctadécylamide avec un rendement de 87 %.

### EXEMPLE 2

On traite pendant 12 heures la L-5-carboxy-tétra-t.butoxycarbonylspermine (1 équivalent) par la N-hydroxysuccinimide (1,1 équivalent) en présence de dicyclohexylcarbodiimide (1,1 équivalent) en opérant dans un mélange chlorure de méthylène-tétrahydrofuranne.

L'ester obtenu est traité par la dipalmitoylphosphatidyléthanolamine (1 équivalent) en présence de triéthylamine (1 équivalent) dans un mélange chloroforme-éthanol pendant 12 heures à 40°C. Après traitement du mélange réactionnel, on obtient, avec un rendement de 55 %, la tétra-t.butoxycarbonyl-5-carboxyspermylamide de la dipalmitoylphosphatidyléthanolamine dont les groupements protecteurs sont éliminés par l'acide trifluoroacétique dans le chlorure de méthylène. On obtient ainsi la 5-carboxyspermylamide de la dipalmitoylphosphatidyléthanolamine (DPPES) sous forme de tétratrifluoroacétate.

La structure du produit obtenu est confirmée par le spectre de résonance magnétique nucléaire du proton à 200 MHz dans le mélange chloroforme deutéré-méthanol deutéré (1-1 en volumes) (déplacements chimiques δ en ppm : 0,85 [t, (CH₃)₂] ; [m, 2 x (CH₂)₁₂] ; 1,5-1,65 (m, 2 x CH₂CO₂) ; 1,8-2,1 (m, 4 x CH₂CH₂N⁺) ; 2,3 (tt, 2 x CH₂CH₂CO₂) ; 2,9-3,1 (m, 5 x CH₂N⁺) ; 3,2 (bm, CH₂NDCO) ; 3,75-4,05 (m, CHN⁺, 2 x CH₂OP) ; 4,15-4,40 (2 x dd, CO₂CH₂) ; 5,20 (OCH).

### EXEMPLE 3

Une solution 20mM de DOGS dans l'éthanol est diluée 10 fois par de l'eau stérile de façon à obtenir une solution 2mM. On prélève 7,5 µl de cette solution que l'on dilue dans 250 µl de milieu DMEM (Dulbecco Modified Essential Medium).

On prépare une solution contenant 5 µg de plasmide contenant un vecteur d'expression de la chloramphénicol acétyl transférase CAT (par exemple une construction dérivée du plasmide pCAT 8+ [L. Klein-Hitpass et coll., Cell, 46, 1053-1061 (1986)] par insertion d'un fragment (BamHI-XbaI) contenant 4 copies de la séquence AP1 ["binding consensus sequence" (pCAT 4XB)] dans 250 µl de milieu DMEM.

On prépare une suspension de 10⁵-10⁶ cellules mélanotropes [préparées selon B.A. Demeineix et coll., Neuroscience, 17, 1275-1285 (1986)] dans 500 µl de milieu DMEM en l'absence de sérum.

Les solutions de DOGS et de plasmide sont mélangées et le mélange "transfectant" est ajouté à la suspension de cellules.

Le mélange est incubé à 37°C pendant une période déterminée.

Après transfection, les cellules sont lavées deux fois puis étalées. Après 48 heures, les cellules sont lavées par un tampon phosphate (PBS) puis on détermine l'activité chloramphénicol acétyl transférase selon la méthode de C.M. Gorman et coll., Mol. Cell. Biol., 2, 1044-1051 (1982).

Les cellules sont remises en suspension dans 100 µl d'une solution 200mM de TRIS-HCl (pH = 7,4). Après plusieurs cycles de refroidissement-réchauffement, 50 µl du surnageant sont ajoutés à 40 µl de TRIS-HCl (pH = 7,4) contenant du chloramphénicol marqué au ¹⁴C (0,1 µCi). Après 5 minutes à 37°C, la réaction est initiée par addition de 20 µl d'acétyl CoA (4mM). Après 1 heure à 37°C, le chloramphénicol et ses dérivés acétylés sont extraits à l'acétate d'éthyle, séparés par chromatographie en couche mince et autoradiographiés. Les autoradiogrammes sont analysés par une méthode appropriée.

Cette méthode permet ainsi l'analyse de différents promoteurs dans les cultures primaires en général.

## Revendications

1. Nouvelle lipopolyamine sous forme D, L ou DL caractérisée en ce qu'elle répond à la formule générale : 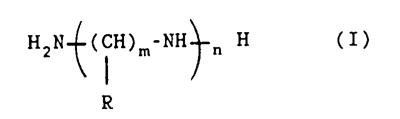 dans laquelle :
- n est un nombre entier compris entre 1 et 5 inclusivement,
- m est un nombre entier compris entre 2 et 6 inclusivement
- R représente un atome d'hydrogène ou un radical de formule générale: 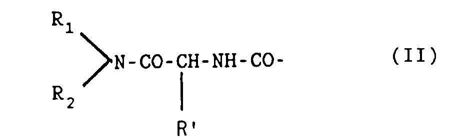 dans lequel R₁ et R₂, identiques ou différents, représentent chacun un radical aliphatique saturé CₚH₂ₚ₊₂ ou insaturé CₚH₂ₚ ou CₚH₂ₚ₋₂, P étant un nombre entier compris entre 12 et 22 inclusivement, et R' représente un atome d'hydrogène ou un radical alkyle contenant 1 à 4 atomes de carbone éventuellement substitué par un radical phényle, ou un radical de formule générale : 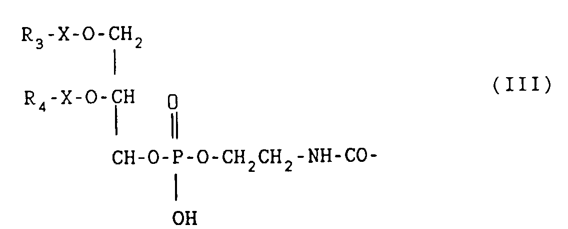 dans laquelle X représente un groupement méthylène ou un groupement carbonyle et R₃ et R₄, identiques ou différents, représentent chacun un radical aliphatique saturé C_{p'}H_{2p'+2} ou insaturé C_{p'}H_{2p'} ou C_{p'}H_{2p'-2}, p' étant un nombre entier compris entre 11 et 21 inclusivement, étant entendu que :
- quelles que soient les valeurs de m et n, un et un seul des symboles R représente un radical de formule générale (II) ou (III)
- lorsque n est compris entre 2 et 5, les valeurs de m dans les différents fragments 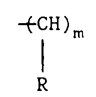 peuvent être identiques ou différentes, ainsi que ses sels.

2. Nouvelle lipopolyamine selon la revendication 1 caractérisée en ce que n est égal à 3 et les valeurs de m dans les fragments 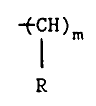 sont identiques ou différentes et représentent 3 ou 4 et R représente soit un radical de formule générale (II) dans lequel R₁ et R₂ représentent chacun un radical alkyle contenant 11 à 22 atomes de carbone et R' représente un atome d'hydrogène soit un radical de formule générale (III) dans lequel R₃-X- et R₄-X-représentent chacun un radical alcanoyle contenant 12 à 22 atomes de carbone, et ses sels.

3. La 5-carboxyspermylglycine dioctadécylamide.

4. La 5-carboxyspermylamide de la dipalmitoylphosphatidyléthanolamine.

5. Procédé de préparation d'une lipopolyamine selon la revendication 1 pour laquelle R représente un radical de formule générale (II) caractérisé en ce que l'on fait agir un produit de formule générale : 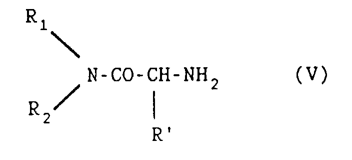 dans laquelle R₁, R₂ et R' sont définis comme dans la revendication 1, sur un produit de formule générale : 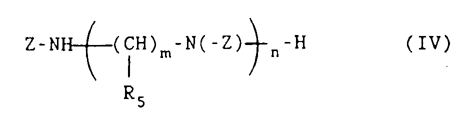 dans laquelle m et n sont définis comme dans la revendication 1, R₅ représente un atome d'hydrogène ou un radical carboxy et les symboles Z représentent un groupement protecteur de la fonction amine, étant entendu que :
- quelles que soient les valeurs de m et n, un et un seul des symboles R₅ représente un radical carboxy
- lorsque n est compris entre 2 et 5, les valeurs de m des différents fragments 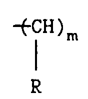 peuvent être identiques ou différentes,
en présence d'un diimide dans un solvant organique inerte choisi parmi les hydrocarbures aliphatiques halogénés, puis remplace les groupements protecteurs Z par un atome d'hydrogène, et isole le produit obtenu éventuellement sous forme de sel.

6. Procédé de préparation d'une lipopolyamine selon la revendication 1 pour laquelle R représente un radical de formule générale (III) caractérisé en ce que l'on fait réagir un produit de formule générale : 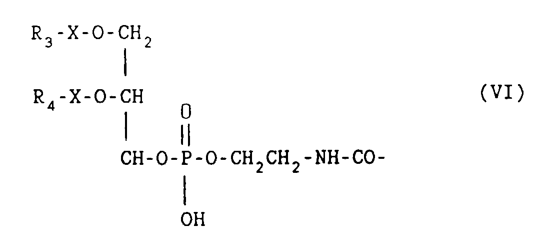 dans laquelle R₃, R₄ et X sont définis comme dans la revendication 1, avec un ester avec le N-hydroxysuccinimide du produit de formule générale : 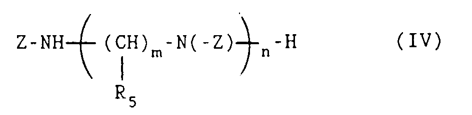 dans laquelle m, n, R₅ et Z sont définis comme dans la revendication 5 en opérant dans un solvant organique choisi parmi les hydrocarbures aliphatiques chlorés et les alcools en présence d'une base organique choisie parmi les amines tertiaires, puis remplace les groupements protecteurs Z par un atome d'hydrogène, et isole le produit obtenu éventuellement sous forme de sel.

7. Utilisation d'une lipopolyamine selon l'une des revendications 1 à 4 pour la transfection de cellules eucaryotes caractérisée en ce l'on mélange dans un milieu convenable une solution aqueuse diluée d'un produit selon l'une des revendications 1 à 4 avec une solution aqueuse diluée de l'ADN plasmidique ou oligonucloétidique puis met en contact le mélange transfectant avec une suspension de cellules à transfecter.

8. Utilisation selon la revendication 7 caractérisée en ce que l'on transfecte des lignées cellulaires ou des cultures primaires.

9. Une solution éthanolique d'un produit selon l'une des revendications 1 à 4 pour réaliser la transfection selon l'une des revendications 7 ou 8.

10. Une solution aqueuse d'un produit selon l'une des revendications 1 à 4 pour réaliser la transfection selon l'une des revendications 7 ou 8.

11. Composé de formule générale : 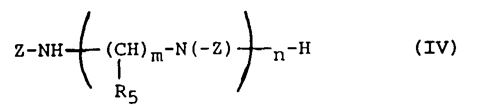 dans laquelle n est un nombre entier compris entre 2 et 5 inclusivement m est un nombre entier compris entre 2 et 6 inclusivement, R₅ représente un atome d'hydrogène ou un radical carboxy et les symboles Z représentent un groupement protecteur de la fonction amine, étant entendu que:
- quelles que soient les valeurs de m et n, un et un seul des symboles R₅ représente un radical carboxy et
- les valeurs de m des différents fragments 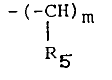 peuvent être identiques ou différentes.

## Patentansprüche

1. Neues Lipopolyamin in D-, L- oder DL-Form, dadurch gekennzeichnet, daß es der allgemeinen Formel : 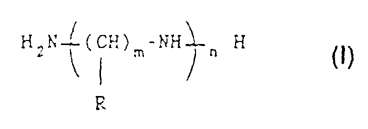 entspricht, in welcher:
- n eine ganze Zahl zwischen einschließlich 1 und 5 ist,
- m eine ganze Zahl zwischen einschließlich 2 und 6 ist,
- R ein Wasserstoffatom oder einen Rest der allgemeinen Formel: 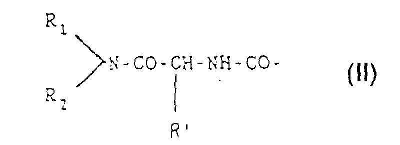 darstellt, in welchem R₁ und R₂ gleich oder verschieden sind und jeweils einen aliphatischen gesättigten CₚH₂ₚ₊₂- oder ungesättigten CₚH₂ₚ- oder CₚH₂ₚ₋₂-Rest darstellen, wobei p eine ganze Zahl zwischen einschließlich 12 und 22 ist und R' ein Wasserstoffatom oder einen gegebenenfalls mit einem Phenylrest substituierten Alkylrest mit 1 bis 4 Kohlenstoffatomen darstellt, oder einen Rest der allgemeinen Formel: 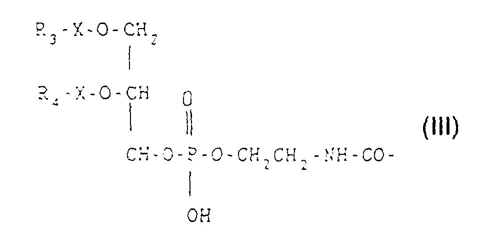 darstellt, in welcher X eine Methylengruppe oder eine Carbonylgruppe darstellt und R₃ und R₄ gleich oder verschieden sind und jeweils einen aliphatischen gesättigten C_{p'}H_{2p'+2}- oder ungesättigten C_{p'}H_{2p'}- oder C_{p'}H_{2p'-2}-Rest darstellen, wobei p' eine ganze Zahl zwischen einschließlich 11 und 21 ist, wobei es sich versteht, daß,
- wie auch immer die Werte von m und n sind, eines und nur eines der Symbole R einen Rest der allgemeinen Formel (II) oder (III) darstellt,
- wenn n zwischen 2 und 5 liegt, die Werte von m in den verschiedenen Fragmenten 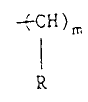 gleich oder verschieden sein können,
sowie seine Salze.

2. Neues Lipopolyamin gemäß Anspruch 1, dadurch gekennzeichnet, daß n gleich 3 ist und die Werte von m in den Fragmenten 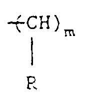 gleich oder verschieden sind und 3 oder 4 darstellen und R entweder einen Rest der allgemeinen Formel (II), in welchem R₁ und R₂ jeweils einen Alkylrest mit 11 bis 22 Kohlenstoffatomen darstellen und R' ein Wasserstoffatom darstellt, oder einen Rest der allgemeinen Formel (III), in welchem R₃-X- und R₄-X- jeweils einen Alkanoylrest mit 12 bis 22 Kohlenstoffatomen darstellen, darstellt, und seine Salze.

3. 5-Carboxyspermylglycin-dioctadecylamid.

4. Dipalmitoylphosphatidylethanolamin-5-Carboxyspermylamid.

5. Verfahren zur Herstellung eines Lipopolyamins gemäß Anspruch 1, für welches R einen Rest der allgemeinen Formel (II) darstellt, dadurch gekennzeichnet, daß man ein Produkt der allgemeinen Formel: 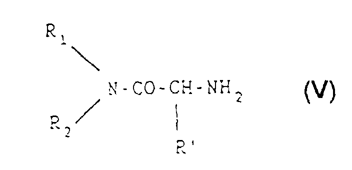 in welcher R₁, R₂ und R' wie in Anspruch 1 definiert sind, auf ein Produkt der allgemeinen Formel: 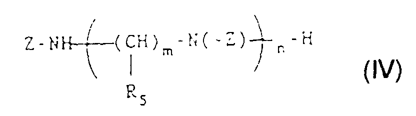 in welcher m und n wie in Anspruch 1 definiert sind, R₅ ein Wasserstoffatom oder einen Carboxyrest darstellt und die Symbole Z eine Schutzgruppe für die Aminfunktion darstellen, wobei es sich versteht, daß
- wie auch immer die Werte von m und n sind, eines und nur eines der Symbole R₅ einen Carboxyrest darstellt,
- wenn n zwischen 2 und 5 liegt, die Werte von m der verschiedenen Fragmente 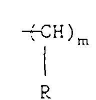 gleich oder verschieden sein können,
in Gegenwart eines Diimids in einem inerten organischen Lösungsmittel, das unter den halogenierten aliphatischen Kohlenwasserstoffen ausgewählt ist, wirken läßt, dann die Schutzgruppen Z durch ein Wasserstoffatom ersetzt und das erhaltene Produkt, gegebenenfalls in Salzform, isoliert.

6. Verfahren zur Herstellung eines Lipopolyamins gemäß Anspruch 1, für welches R einen Rest der allgemeinen Formel (III) darstellt, dadurch gekennzeichnet, daß man ein Produkt der allgemeinen Formel: 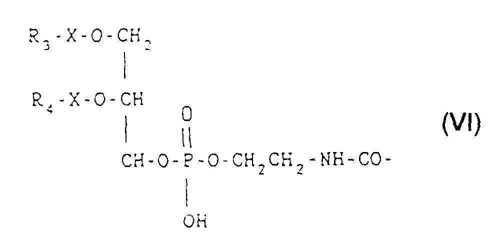 in welcher R₃, R₄ und X wie in Anspruch 1 definiert sind, mit einem Ester mit dem N-Hydroxysuccinimid des Produkts der allgemeinen Formel: 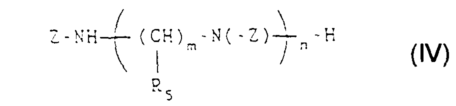 in welcher m, n, R₅ und Z wie in Anspruch 5 definiert sind, umsetzt, wobei man in einem organischen Lösungsmittel, das unter den chlorierten aliphatischen Kohlenwasserstoffen und den Alkoholen ausgewählt ist, in Gegenwart einer organischen Base, die unter den tertiären Aminen ausgewählt ist, arbeitet, dann die Schutzgruppen Z durch ein Wasserstoffatom ersetzt und das erhaltene Produkt, gegebenenfalls in Salzform, isoliert.

7. Verwendung eines Lipopolyamins gemäß einem der Ansprüche 1 bis 4 für die Transfektion von Eukaryontenzellen, dadurch gekennzeichnet, daß man in einem geeigneten Medium eine verdünnte wäßrige Lösung eines Produkts gemäß einem der Ansprüche 1 bis 4 mit einer verdünnten wäßrigen Lösung von plasmidischer oder oligonucleotidischer DNS mischt, dann das Transfektionsgemisch mit einer Suspension von zu transfektierenden Zellen in Kontakt bringt.

8. Verwendung gemäß Anspruch 7, dadurch gekennzeichnet, daß man Zellstämme oder Primärkulturen transfektiert.

9. Eine ethanolische Lösung eines Produkts gemäß einem der Ansprüche 1 bis 4 zur Durchführung der Transfektion gemäß einem der Ansprüche 7 oder 8.

10. Eine wäßrige Lösung eines Produkts gemäß einem der Ansprüche 1 bis 4 zur Durchführung der Transfektion gemäß einem der Ansprüche 7 oder 8.

11. Verbindung der allgemeinen Formel: 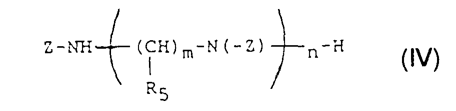 in welcher n eine ganze Zahl zwischen einschließlich 2 und 5 ist, m eine ganze Zahl zwischen einschließlich 2 und 6 ist, R₅ ein Wasserstoffatom oder einen Carboxyrest darstellt und die Symbole Z eine Schutzgruppe für die Aminfunktion darstellen, wobei es sich versteht, daß,
- wie auch immer die Werte von m und n sind, eines und nur eines der Symbole R₅ einen Carboxyrest darstellt und
- die Werte von m der verschiedenen Fragmente den sein können. 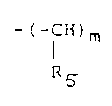 gleich oder verschieden sein können.

## Claims

1. New lipopolyamine in the D, L or DL form, characterized in that it corresponds to the general formula: 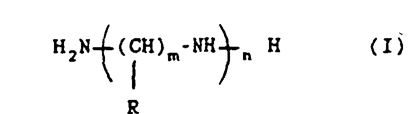 in which:
- n is an integer between 1 and 5 inclusive,
- m is an integer between 2 and 6 inclusive,
- R represents a hydrogen atom or a radical of general formula: 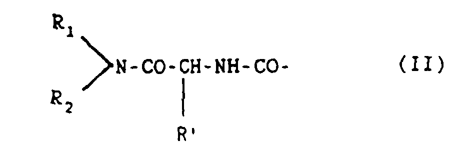 in which R₁ and R₂, which are identical or different, each represent a saturated CₚH₂ₚ₊₂ or unsaturated CₚH₂ₚ or CₚH₂ₚ₋₂ aliphatic radical, p being an integer between 12 and 22 inclusive, and R' represents a hydrogen atom or an alkyl radical containing 1 to 4 carbon atoms, optionally substituted by a phenyl radical, or a radical of general formula: 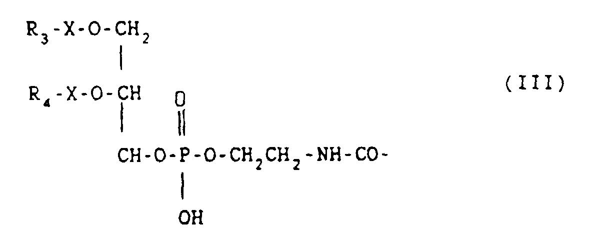 in which X represents a methylene group or a carbonyl group and R₃ and R₄, which are identical or different, each represent a saturated C_{p'}H_{2p'+2} or unsaturated Cₚ,H_{2p'} or C_{p'}H_{2p'-2} aliphatic radical, p' being an integer between 11 and 21 inclusive, it being understood that:
- whatever the values of m and n, one and only one of the symbols R represents a radical of general formula (II) or (III)
- when n is between 2 and 5, the values of m in the different fragments 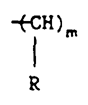 may be identical or different,
as well as its salts.

2. New lipopolyamine according to Claim 1, characterized in that n is equal to 3 and the values of m in the fragments 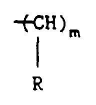 are identical or different and represent 3 or 4 and R represents either a radical of general formula (II) in which R₁ and R₂ each represent an alkyl radical containing 11 to 22 carbon atoms and R' represents a hydrogen atom or a radical of general formula (III) in which R₃-X- and R₄-X- each represent an alkanoyl radical containing 12 to 22 carbon atoms, and its salts.

3. Dioctadecylamide 5-carboxyspermylglycine.

4. Dipalmitoylphosphatidylethanolamine 5-carboxyspermylamide.

5. Process for the preparation of a lipopolyamine according to Claim 1 for which R represents a radical of general formula (II), characterized in that a product of general formula: 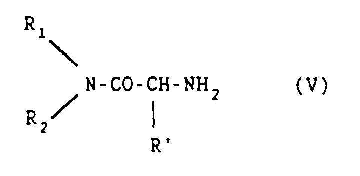 in which R₁, R₂ and R' are defined as in Claim 1, is reacted with a product of general formula: 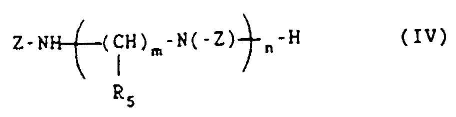 in which m and n are defined as in Claim 1, R₅ represents a hydrogen atom or a carboxyl radical and the symbols Z represent a group for protecting the amine functional group, it being understood that:
- whatever the values of m and n, one and only one of the symbols R₅ represents a carboxyl radical
- when n is between 2 and 5, the values of m for the different fragments 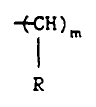 may be identical or different,
in the presence of a diimide in an inert organic solvent chosen from halogenated aliphatic hydrocarbons, and then the protecting groups Z are replaced by a hydrogen atom, and the product obtained is isolated optionally in the form of a salt.

6. Process for the preparation of a lipopolyamine according to Claim 1 for which R represents a radical of general formula (III), characterized in that a product of general formula: 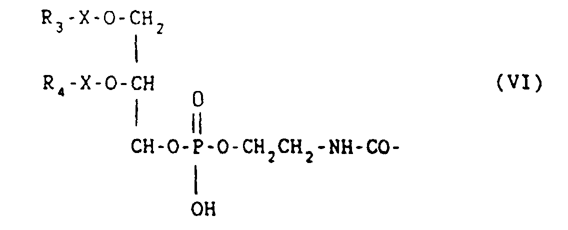 in which R₃, R₄ and X are defined as in Claim 1, is reacted with an ester with the N-hydroxysuccinimide of the product of general formula: 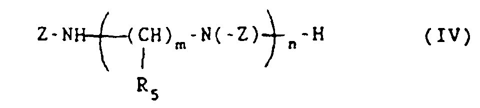 in which m, n, R₅ and Z are defined as in Claim 5, the procedure being carried out in an organic solvent chosen from chlorinated aliphatic hydrocarbons and alcohols in the presence of an organic base chosen from the tertiary amines, and then the protecting groups Z are replaced by a hydrogen atom, and the product obtained is isolated optionally in the form of a salt.

7. Use of a lipopolyamine according to one of Claims 1 to 4 for the transfection of eukaryotic cells, characterized in that a dilute aqueous solution of a product according to one of Claims 1 to 4 is mixed in a suitable medium with a dilute aqueous solution of oligonucleotide or plasmid DNA and then the transfectant mixture is brought into contact with a suspension of cells to be transfected.

8. Use according to Claim 7, characterized in that primary cultures or cell lines are transfected.

9. Ethanolic solution of a product according to one of Claims 1 to 4, for carrying out the transfection according to either of Claims 7 and 8.

10. Aqueous solution of a product according to one of Claims 1 to 4, for carrying out the transfection according to either of Claims 7 and 8.

11. Compound of general formula: 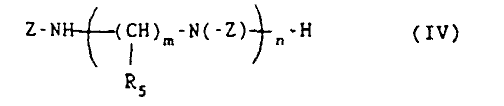 in which n is an integer between 2 and 5 inclusive, m is an integer between 2 and 6 inclusive, R₅ represents a hydrogen atom or a carboxyl radical and the symbols Z represent a group for protecting the amine functional group, it being understood that:
- whatever the values of m and n, one and only one of the symbols R₅ represents a carboxyl radical, and
- the values of m for the different fragments 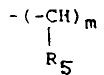 may be identical or different.
